# FASCICULE DE BREVET EUROPEEN

(11) **EP 3 648 809 B1**
(45) Date de publication et mention de la délivrance du brevet: **29.10.2025**
(21) Numéro de dépôt: 18745836.9
(22) Date de dépôt: 06.07.2018
(51) Int. Cl.: A61L 29/04, C08F 293/00, C08L 53/00, A61L 31/04

(54) **MATERIAU SOLIDE ORGANIQUE ANTIBACTERIEN**
FESTES ORGANISCHES ANTIBAKTERIELLES MATERIAL
SOLID ORGANIC ANTIBACTERIAL MATERIAL

(30) Priorité: 06.07.2017 FR 1756390
(43) Date de publication de la demande: 13.05.2020
(73) Titulaire: Centre National de la Recherche Scientifique, 75794 Paris Cedex 16 (FR); Université d'Aix Marseille, 13007 Marseille (FR); Ecole Centrale de Marseille, 13013 Marseille (FR)
(72) Inventeur: GIGMES, Didier, 13190 Allauch (FR); GUILLANEUF, Yohann, 13013 Marseille (FR); GUILLANEUF, Catherine, 13013 Marseille (FR); MARESCA, Marc, René, Sauveur, 13127 Vitrolles (FR); YSACCO, Cédric, 37520 La Riche (FR)
(74) Mandataire: Osha BWB
(86) Numéro de dépôt international: PCT/EP2018/068449
(87) Numéro de publication internationale: WO 2019/008176

(56) Documents cités:
- EP-A2- 0 344 692
- US-A1- 2005 147 647
- MARTA ÁLVAREZ-PAINO ET AL: "Effect of glycounits on the antimicrobial properties and toxicity behavior of polymers based on quaternized DMAEMA", BIOMACROMOLECULES, vol. 16, no. 1, 10 December 2014 (2014-12-10), US, pages 295 - 303, XP055475667, ISSN: 1525-7797, DOI: 10.1021/bm5014876
- THOMAS BREINER ET AL: "Blends of Poly(methacrylate) Block Copolymers with Photoaddressable Segments", MACROMOLECULES, vol. 40, no. 6, 1 March 2007 (2007-03-01), US, pages 2100 - 2108, XP055475878, ISSN: 0024-9297, DOI: 10.1021/ma0624907
- YANQIANG WANG ET AL: "Antimicrobial and Hemolytic Activities of Copolymers with Cationic and Hydrophobic Groups: A Comparison of Block and Random Copolymers", MACROMOLECULAR BIOSCIENCE, 4 August 2011 (2011-08-04), DE, pages n/a - n/a, XP055475592, ISSN: 1616-5187, DOI: 10.1002/mabi.201100196
- BELKACEM TAREK BENKHALED ET AL: "Elaboration of antimicrobial polymeric materials by dispersion of well-defined amphiphilic methacrylic SG1-based copolymers", POLYMER CHEMISTRY, vol. 9, no. 22, 1 January 2018 (2018-01-01), GB, pages 3127 - 3141, XP055509571, ISSN: 1759-9954, DOI: 10.1039/C8PY00523K

## Description

### DOMAINE TECHNIQUE

La présente description concerne des copolymères amphiphiles à blocs du type méthacrylique et des matériaux solides organiques ; leur procédé de préparation ; et leur utilisation pour application antibactérienne, antimicrobienne, antiinflammatoire et/ou antifongique.

### ETAT DE L'ART

La résistance microbienne aux antibiotiques est devenue un enjeu de santé publique. Certains antibiotiques comme la méticilline ou la vancomycine sont en effet devenus inefficaces pour lutter contre des infections provenant de bactéries comme les Staphylococus aureus résistant à la méticilline ou la vancomycine (MRSA ou VRSA respectivement). Les matériaux antibactériens peuvent donc jouer un rôle dans la lutte contre les infections bactériennes, et ce plus particulièrement dans le domaine hospitalier où les infections sont notamment une des causes principales de complications dans les services de réanimation.

Différentes méthodes existent aujourd'hui pour élaborer des matériaux antibactériens : i) l'inclusion de molécules biocides (antibiotiques ou molécules à base d'argent par exemple) mais qui sont relarguées hors du matériau, conduisant à une perte d'activité du matériau, dans certains cas des effets secondaires et/ou une exacerbation du phénomène de résistance antimicrobienne ; ii) la modification de surface du matériau par greffage ou couplage, qui est une méthode longue, couteuse et multi-étapes ; iii) l'utilisation de surfactants antibactériens (à hauteur d'environ 10 % en poids) pour réaliser des films antimicrobiens.

Depuis quelques années, les peptides antimicrobiens (appelés AMP pour « antimicrobial peptides ») font l'objet de nombreuses études pour tenter d' offrir une alternative aux antibiotiques actuels. Contrairement aux antibiotiques classiques, les AMP présentent en effet l'avantage de ne pas induire de résistance grâce à un mécanisme d'action différent touchant principalement la membrane des bactéries (Michl et al. Polym. Chem. 2014, 19, 5813). Cependant, le principal frein à l'utilisation des AMP est leur cout et leur faible volume de production, les peptides étant soit obtenus par synthèse peptidique sur phase solide dans le cas des peptides synthétiques soit extraits de différentes sources naturelles comme des venins par exemple. Pour pallier à ces problèmes, divers copolymères synthétiques ont été étudiés pour mimer l'activité antimicrobienne des AMP mais tout en étant facilement synthétisables et ce à grande échelle. Ces copolymères, appelés SMAMP pour « synthetic mimic antimicrobial peptides », sont en général des copolymères amphiphiles présentant une partie hydrophile, le plus souvent chargée, venant interagir avec la membrane des bactéries pour s'y accrocher et une partie hydrophobe venant déstabiliser ou percer la membrane des bactéries (Kuroda et al. Nanomed. Nanobiotechnology 2013, 5, 49 ; Takahashi et al. Macromol. Biosci. 2013, 13, 1285). Si le mode d'action des SMAMP reproduit celui des AMP, leur synthèse est par contre beaucoup plus aisée car réalisée par différentes techniques de polymérisation et en particulier par polymérisation radicalaire qui permet une production à très grande échelle.

Par ailleurs, Lenoir et al. (Biomacromolecules, 2006, 7, 2291) ont rapporté en 2006 la dispersion de copolymères dibloc poly(éthylène-*co*-butylène)-b-poly(2-(*tert-*butylamino)éthyl méthacrylate) (PEB-*b*-PTBAEMA, *M*ₙ = 14000 g/mol) pour conférer des propriétés antimicrobiennes à des films de polyéthylène basse densité (PEBD). Les copolymères ont été mélangés dans du PEBD à hauteur de 10 % en poids et ont montré une activité contre *E. coli* (Gram -). Zuo et al. (Journal of Applied Polymer Science, 2012, 125, 3537) ont rapporté pour leur part l'élaboration de poly(méthacrylate de méthyle) (PMMA) commercial rendu antibactérien par mélange avec au minimum 10 % en poids de poly(t*ert-*butylamino)éthyl méthacrylate) (PTBAEMA, *M_{w}* ≤ 10000 g/mol) pour monter une activité contre *S. aureus* (Gram +) et *E. coli* (Gram -). Enfin, Li et al. (European Polymer Journal, 2014, 51, 120) ont montré qu'il était possible de préparer du polypropylène (PP) antibactérien par greffage en CO₂ supercritique et par extrusion réactive de chaînes de poly(hexaméthylènediamine-chlorure de guanidinium). Ce procédé complexe a permis d'élaborer des matériaux à base de PP actifs vis à vis des bactéries du type *S. aureus* et *E. coli.*

Cependant, le copolymère utilisé est typiquement de faible masse molaire (< 15000 g/mol) pour imiter la structure des peptides antimicrobiens naturel. D'après la littérature, une masse molaire plus élevée s'accompagnerait aussi d'une augmentation du caractère hémolytique du copolymère (Ganewatta et al. Polymer 2015, 63, A1). De plus, la quantité de copolymère nécessaire pour observer une activité antimicrobienne est élevée, autour des 10 % en poids. L'activité reportée ne concerne par ailleurs que des souches bactériennes modèles (typiquement *S. aureus* et *E. coli*) qui n'incluent pas les souches pathogènes pourtant d'un enjeu sanitaire crucial pour demain. Enfin, les différents procédés d'élaboration cités précédemment ne permettent d'élaborer des matériaux organiques antibactériens que d'une seule nature chimique donnée (du PEHB ou du PMMA ou du PP). Un procédé d'incorporation versatile du copolymère antimicrobien dans des matrices organiques de nature chimique différente fait donc défaut. En conséquence, il existe un besoin de matériaux solides organiques et leur procédé de préparation permettant de surmonter certaines des limitations citées ci-dessus.

### RESUME

La présente description a pour objet de fournir des copolymères amphiphiles à blocs du type méthacrylique et des matériaux solides organiques à propriétés antibactériennes, antimicrobiennes, antiinflammatoires et/ou antifongiques.

Selon un premier aspect l'objet précité, ainsi que d'autres avantages, sont obtenus par un matériau solide organique comprenant de 0,02 à 2 % en poids d'au moins un copolymère amphiphile à blocs du type méthacrylique, le copolymère à blocs étant dispersé dans une matrice polymérique, le copolymère à blocs présentant une masse molaire moyenne en nombre (*M*ₙ) supérieure ou égale à 20 000 g/mol. Le copolymère à blocs comprend au moins un bloc méthacrylique hydrophile comprenant au moins un groupe amine tertiaire et au moins un groupe ion ammonium quaternaire.

Selon un ou plusieurs modes de réalisation, le copolymère à blocs présente une *M*ₙ inférieure ou égale à 100 000 g/mol. Selon un ou plusieurs modes de réalisation, le copolymère à blocs présente une masse molaire moyenne en nombre *(Mn)* comprise entre 20 000 et 70 000 g/mol. Selon un ou plusieurs modes de réalisation, le copolymère à blocs présente une masse molaire moyenne en nombre *(Mn)* comprise entre 21 000 et 55 000 g/mol. Selon un ou plusieurs modes de réalisation, le copolymère à blocs présente une masse molaire moyenne en nombre *(Mn)* comprise entre 22 000 et 40 000 g/mol.

Selon un ou plusieurs modes de réalisation, le copolymère à bloc est un polymère dibloc, *i*.*e*., comprenant deux types de monomères.

Le matériau solide organique comprend de 0.02 à 2 % en poids (wt%) du copolymère à blocs. Selon un ou plusieurs modes de réalisation, le matériau solide organique comprend de 0.1 à 2 % en poids du copolymère à blocs. Selon un ou plusieurs modes de réalisation, le matériau solide organique comprend de 0.2 à 2 % en poids du copolymère à blocs.

Selon un ou plusieurs modes de réalisation, le copolymère à blocs présente au moins une partie hydrophile, *i*.*e*., au moins un bloc méthacrylique hydrophile, et au moins une partie hydrophobe, *i.e.,* au moins un bloc méthacrylique hydrophobe.

Le terme « hydrophile » est synonyme de (signifie la même chose que) étant susceptible de former des liaisons hydrogène, tel qu'avec un solvant polaire, tel que de l'eau. Le terme « hydrophobe » est synonymes de (signifie la même chose que) n' étant pas susceptible de former des liaisons hydrogène, tel qu'avec un solvant polaire, tel que de l'eau.

Le bloc méthacrylique hydrophile comprend au moins un groupe amine tertiaire et au moins un groupe ion ammonium quaternaire.

Selon un ou plusieurs modes de réalisation, le monomère méthacrylique hydrophile (*i.e.,* motif de répétition hydrophile du bloc méthacrylique hydrophile) est choisi parmi le groupe constitué par un méthacrylate de *N,N*-(dialkylamino)alkyle, tel qu'un méthacrylate de N,N-(dialkylamino)éthyle, et un ion ammonium quaternaire de celui-ci. Selon un ou plusieurs modes de réalisation, au moins une des fonctions alkyle est un alkyle substitué ou non substitué et/ou comprend entre 1 et 8 atomes de carbones, tel que entre 2 et 6 atomes de carbones.

Le terme « non substitué » est synonymes de (signifie la même chose que) non substitué d'un atome autre que un ou plusieurs atomes d'hydrogène.

Le terme « substitué » est synonyme de (signifie la même chose que) substitué d'au moins un élément autre qu'un atome d'hydrogène, par exemple substitué d'au moins un substituant hydrocarboné. Selon un ou plusieurs modes de réalisation, l'élément est choisi parmi le groupe constitué par un alkyle, un alcényle, un alcynyle, un aryle, un hétéroaryle, un alkylalcényle, un alcénylalkyle, un alkylalcynyle, un alcynylalkyle, un alkylaryle, un arylalkyle, un alkylhétéroaryle, un hétéroarylalcényle, un alcénylaryle, un alcénylhétéroaryle, un arylalcynyle, un hétéroarylalcynyle, un alcynylaryle et un alcynylhétéroaryle, l'élément comprenant de 1 à 20 atomes de carbone, tel que de 2 à 18, 3 à 16, 4 à 14 ou 5 à 12 atomes de carbones ; lequel élément comprenant optionnellement un ou plusieurs hétéroatomes, tel que par exemple N, O, S, P, Si, Sn, Ge, As, F, Cl, Br et I ; et/ou lequel élément comprenant optionnellement un ou plusieurs groupes fonctionnels choisis parmi la liste constitué par un alkyle, un alcène, un alcyne, un aryle, un hétéroaryle, un alcool, une cétone, un benzoyle, un aldéhyde, un carbonate, un acide carboxylique, un carboxylate, un ester, un éther-oxyde, un hétérocycle, une amine, une amide, un azo, un diazo, un diazoamino, un azoture, une imine secondaire, une hydrazine, une hydrazone, une amidine, un carbamate, une guanidine, une carbodiimide, un nitrile, un isonitrile, un imide, un azide, un diimide, un thiol, un thioether, une thiocétone, un cyanate, un nitrate, un nitrite, un nitro, un nitroso, une oxime, un pyridyl, un thioether, un disulfide, un sulfinyl, un sulfonyl, un thiocyanate, un isothiocyanate, une thione, un phosphorane, une phosphine, un boronate, un borinate, un silane et un halogène, les groupes fonctionnels comprenant de 0 à 20 atomes de carbones, tel que de 1 à 20, 2 à 18, 3 à 16, 4 à 14 ou 5 à 12 atomes de carbones.

Selon un ou plusieurs modes de réalisation, le monomère méthacrylique hydrophile est choisi parmi le groupe constitué par le méthacrylate de *N,N-(diméthylamino)éthyle* (DMAEMA), le méthacrylate de *N*,*N*-(diéthylamino)éthyle (DEAEMA) et un ion ammonium quaternaire de ceux-ci.

Selon un ou plusieurs modes de réalisation, le monomère méthacrylique hydrophobe (*i.e.,* motif de répétition hydrophobe du bloc méthacrylique hydrophobe) est choisi parmi le groupe constitué par un méthacrylate d'alkyle linéaire, ramifié, cyclique ou cyclique et ramifié, ayant par exemple de 1 à 20 atomes de carbones. Selon un ou plusieurs modes de réalisation, le méthacrylate d'alkyle est choisi parmi le groupe constitué par le méthacrylate de méthyle (MMA) et le méthacrylate de butyle (BMA).

Selon un ou plusieurs modes de réalisation, le bloc méthacrylique hydrophile et/ou le bloc méthacrylique hydrophobe comprend en outre au moins un motif de répétition supplémentaire tel que choisi parmi le groupe constitué par le styrène (S) et l'acrylonitrile (ACN).

Selon un ou plusieurs modes de réalisation, le copolymère à blocs comprend au moins un bloc choisi parmi le PBMA, le PMMA, le PDMAEMA et le PDEAEMA. Selon un ou plusieurs modes de réalisation, le copolymère à blocs comprend au moins une des formulations suivantes : PMMA-*b*-PDMAEMA, PMMA-*b*-PDEAEMA, PBMA-*b-*PDMAEMA, PBMA-*b*-PDEAEMA, P(MMA-*Co*-S)-*b*-PDMAEMA, P(MMA-*Co*-S)-*b-*PDEAEMA, P(BMA-*Co*-S)-*b*-PDMAEMA, P(BMA-*Co*-S)-b-PDEAEMA, P(MMA-*Co-*ACN)-*b*-PDMAEMA, P(MMA-*Co*-ACN)-*b*-PDEAEMA, P(BMA-*Co*-ACN)-*b*-PDMAEMA, P(BMA-*Co*-ACN)-*b*-PDEAEMA, PMMA-*b*-P(DMAEMA-*Co*-S), PMMA-*b*-P(DEAEMA-*Co*-S), PBMA-*b*-P(DMAEMA-*Co*-S), PBMA-*b*-P(DEAEMA-*Co*-S), PMMA-*b*-P(DMAEMA-*Co*-ACN), PMMA-*b*-P(DEAEMA-*Co*-ACN), PBMA-*b*-P(DMAEMA-*Co*-ACN), PBMA-*b-*P(DEAEMA-*Co*-ACN), P(MMA-*Co*-S)-*b*-P(DMAEMA-*Co*-S), P(MMA-Co-S)-*b-*P(DEAEMA-*Co*-S), P(BMA-*C*o-S)-b-P(DMAEMA-*Co*-S), P(BMA-*Co*-S)-*b*-P(DEAEMA-*Co*-S), P(MMA-*Co*-ACN)-*b*-P(DMAEMA-*Co*-ACN), P(MMA-*Co*-ACN)-*b*-P(DEAEMA-*Co-*ACN), P(BMA-*Co*-ACN)-*b*-P(DMAEMA-*Co*-ACN), P(BMA-*Co*-ACN)-*b*-P(DEAEMA-*Co-*ACN), P(MMA-*Co*-S)-*b*-P(DMAEMA-*Co*-ACN), P(MMA-*Co*-S)-*b*-P(DEAEMA-*Co*-ACN), P(BMA-*Co*-S)-*b*-P(DMAEMA-*Co*-ACN), P(BMA-*Co*-S)-*b*-P(DEAEMA-*Co*-ACN), P(MMA-*Co*-ACN)-*b*-P(DMAEMA-*Co*-S), P(MMA-*Co*-ACN)-*b*-P(DEAEMA-*Co*-S), P(BMA-*Co*-ACN)-*b*-P(DMAEMA-*Co*-S) et P(BMA-*Co*-ACN)-*b*-P(DEAEMA-*Co*-S). Selon un ou plusieurs modes de réalisation, le copolymère à blocs comprend une des formulations suivantes : P(BMA-Co-S)-*b*-P(DMAEMA-Co-S), et P(BMA-*Co*-ACN)-*b*-P(DMAEMA-Co-ACN).

Selon un ou plusieurs modes de réalisation, le copolymère à blocs comprend de 20 à 80 % molaire (mol%) de motifs de répétition hydrophiles. Selon un ou plusieurs modes de réalisation, le copolymère à bloc comprend de 50 à 70 % molaire (mol%) de motifs de répétition hydrophiles.

Selon un ou plusieurs modes de réalisation, le copolymère à bloc comprend de 20 à 80 % molaire (mol%) de motifs de répétition hydrophobes. Selon un ou plusieurs modes de réalisation, le copolymère à bloc comprend de 30 à 50 % molaire (mol%) de motifs de répétition hydrophobes.

Selon un ou plusieurs modes de réalisation, le copolymère à bloc comprend de 3 à 20 % en poids (wt%) de motifs de répétition supplémentaires. Selon un ou plusieurs modes de réalisation, le copolymère à bloc comprend de 5 à 10 % en poids (wt%) de motifs de répétition supplémentaires.

Selon un ou plusieurs modes de réalisation, le bloc méthacrylique hydrophobe présente une masse molaire moyenne en nombre (*Mn*) comprise entre 4000 et 30000 g/mol Selon un ou plusieurs modes de réalisation, bloc méthacrylique hydrophobe présente une masse molaire moyenne en nombre (*Mn*) comprise entre 6000 et 20000 g/mol.

Selon un ou plusieurs modes de réalisation, le copolymère à blocs présente une dispersité (D) comprise entre 1 et 2.0. Selon un ou plusieurs modes de réalisation, le copolymère à bloc présente une dispersité (D) comprise entre 1,30 et 1,50.

Selon un ou plusieurs modes de réalisation, le bloc méthacrylique hydrophobe présente une dispersité (D) comprise entre 1,20 et 1,50. Selon un ou plusieurs modes de réalisation, le bloc méthacrylique hydrophobe présente une dispersité (D) comprise entre 1,30 et 1,40.

Selon un ou plusieurs modes de réalisation, la matrice polymérique comprend au moins un motif de répétition choisi parmi le groupe constitué par le polyéthylene (PE), le polypropylène (PP), le polycarbonate (PC), la polyamide (PA), un polyester, le polyurethane (PU), le polystyrène (PS), le poly(méthyl méthacrylate) (PMMA), le poly(chlorure de vinyle) (PVC), résines acryliques, les silicones et les composites thermodurcissables. Des exemples de polyesters comprennent le poly(acide lactique) et le poly(ethylène téréphtalate), etc.

Selon un ou plusieurs modes de réalisation, la matrice polymérique comprend au moins un motif de répétition choisi parmi le groupe constitué par le polystyrène (PS), le poly(méthyl méthacrylate) (PMMA), le polycarbonate (PC).

Selon un deuxième aspect les objets précités, ainsi que d'autres avantages, sont obtenus par un procédé de fabrication d'un matériau solide organique, tel que le matériau solide organique selon le premier aspect, le procédé comprenant la dispersion de 0,02 à 2 % en poids d'au moins un copolymère amphiphile à blocs du type méthacrylique dans une matrice polymérique, dans lequel le copolymère à bloc présente une masse molaire moyenne en nombre (*M*ₙ) supérieure ou égale à 20 000 g/mol. Le copolymère à blocs comprend au moins un bloc méthacrylique hydrophile comprenant au moins un groupe amine tertiaire et au moins un groupe ion ammonium quaternaire.

Selon un ou plusieurs modes de réalisation, le procédé comprend la préparation du copolymère à blocs par polymérisation radicalaire contrôlée par des nitroxydes.

Selon un ou plusieurs modes de réalisation, la polymérisation radicalaire est réalisée en présence du nitroxyde *N*-(2-methylpropyl)-*N*-(1-diethylphosphono-2,2-dimethylpropyl)-N-oxyl) appelé SG1.

Selon un ou plusieurs modes de réalisation, la polymérisation radicalaire est réalisée en présence de l'alcoxyamine (*N*-(2-methylpropyl)-*N*-(1-diethylphosphono-2,2-dimethylpropyl)-O-(2-carboxylprop-2-yl) commercialisée par Arkema sous le nom de BlocBuilder MA.

Selon un ou plusieurs modes de réalisation, la préparation du copolymère amphiphile à blocs du type méthacrylique comprend : la polymérisation d'un des monomères hydrophile ou hydrophobe (optionnellement en présence d'un motif de répétition additionnel) pour former un premier bloc hydrophile ou hydrophobe respectivement ; puis l'extension de ce premier bloc par la polymérisation de l'autre monomère hydrophobe ou hydrophile (optionnellement en présence d'un motif de répétition additionnel) pour former le copolymère à blocs amphiphile hydrophile-bloc-hydrophobe ou hydrophobe-bloc-hydrophile.

Selon un ou plusieurs modes de réalisation, la polymérisation est effectuée par le chauffage des monomères, préférablement sous atmosphère inerte (e.g. sous azote ou argon), en présence d'un amorceur de polymérisation (BlocBuilder MA ou macroalcoxyamine formant le premier bloc du copolymère) et de nitroxyde libre SG1.

Selon un ou plusieurs modes de réalisation, la polymérisation est effectuée à une température comprise entre 80 °C et 130 °C. Selon un ou plusieurs modes de réalisation, la polymérisation est effectuée à une température de 90°C.

Selon un ou plusieurs modes de réalisation, la polymérisation est effectuée pendant une durée comprise entre 4 et 15 h. Selon un ou plusieurs modes de réalisation, la polymérisation est effectuée pendant une durée comprise entre 4 et 10 h, tel que 5h30.

Selon un ou plusieurs modes de réalisation, la polymérisation est effectuée jusqu'à l'obtention d'une conversion comprise entre 30 et 80 % pour chaque bloc (hydrophile et hydrophobe). Selon un ou plusieurs modes de réalisation, la polymérisation est effectuée jusqu'à l'obtention d'une conversion comprise entre 40 et 60 %, tel que entre 45 et 50% pour chaque bloc (hydrophile et hydrophobe).

Selon un ou plusieurs modes de réalisation, la dispersion du copolymère amphiphile à blocs du type méthacrylique dans une matrice polymérique comprend : la préparation d'une solution comprenant un solvant (e.g. THF) et la matrice polymérique (e.g. PS ou PMMA), l'ajout du copolymère à blocs (quaternisé ou pas) à la solution, et l'évaporation du solvant pour obtenir le matériau solide organique.

Selon un ou plusieurs modes de réalisation, le procédé comprenant la quaternisation de l'amine tertiaire. Selon un ou plusieurs modes de réalisation, la quaternisation est effectuée au moyen d'iodure de méthyle (MeI). Selon un ou plusieurs modes de réalisation, le pourcentage de quaternisation de l'amine tertiaire est de 2.0 mol% (par rapport aux chaines de polymère) à 100%, la valeur 100% ne faisant pas partie de l'objet revendiqué.

Selon un troisième aspect, les objets précités, ainsi que d'autres avantages, sont obtenus par une utilisation d'un matériau solide organique selon l'une quelconque des formes de réalisation du premier aspect pour applications non-thérapeutiques, qu'elles soient antibactérienne et/ou antimicrobienne et/ou antifongique.

Selon un quatrième aspect, les objets précités, ainsi que d'autres avantages, sont obtenus par un dispositif comprenant un matériau solide organique selon l'une quelconque des formes de réalisation du premier aspect.

Selon un ou plusieurs modes de réalisation, le dispositif est choisi parmi le groupe constitué par un cathéter.

Selon un aspect supplémentaire, les objets précités, ainsi que d'autres avantages, sont obtenus par un copolymère amphiphile à blocs du type méthacrylique présentant une masse molaire moyenne en nombre *(Mn)* supérieure ou égale à 20 000 g/mol, par exemple pour application antibactérienne et/ou antimicrobienne et/ou anti-inflammatoire et/ou antifongique. Le copolymère amphiphile à blocs du type méthacrylique présente les caractéristiques comme définies ci-dessus lorsque faisant partie du matériau solide organique. Notamment, le copolymère à blocs comprend au moins un bloc méthacrylique hydrophile comprenant au moins un groupe amine tertiaire et au moins un groupe ion ammonium quaternaire. Il est également envisagé que le copolymère amphiphile à blocs du type méthacrylique puisse être utilisé comme médicament.

Selon un aspect supplémentaire, les objets précités, ainsi que d'autres avantages, sont obtenus par un matériau organique selon l'une quelconque des formes de réalisation du premier aspect pour son utilisation en tant que médicament, par exemple antibactérien et/ou antifongique et/ou antiinflammatoire.

Des modes de réalisation selon les aspects référencés ci-dessus ainsi que des avantages supplémentaires apparaîtront à la lecture de la description illustrée par les Figures suivantes et des revendications annexées.

### BREVE DESCRIPTION DES DESSINS

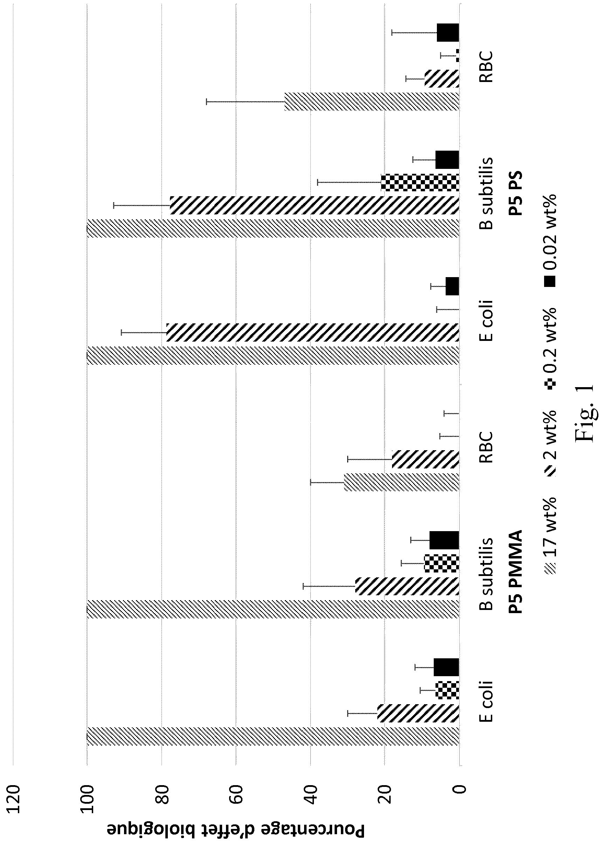

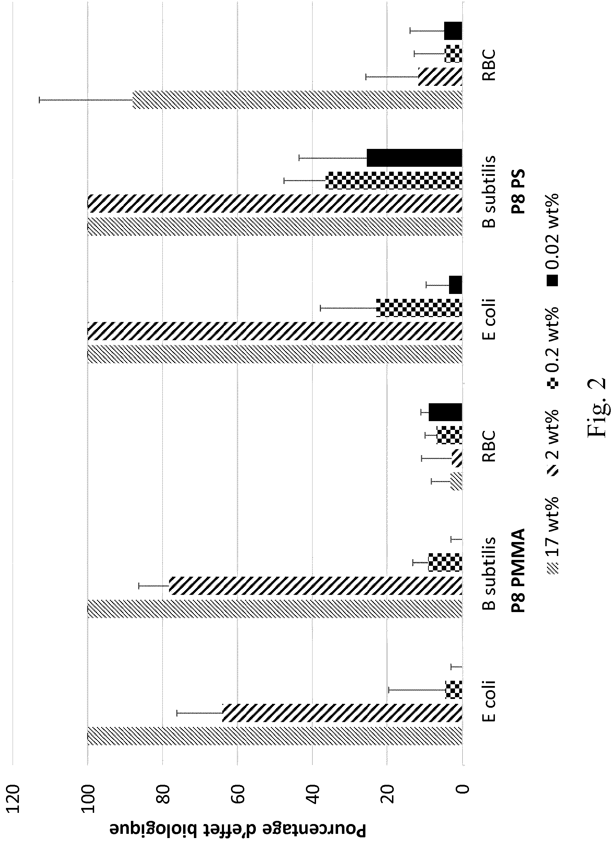

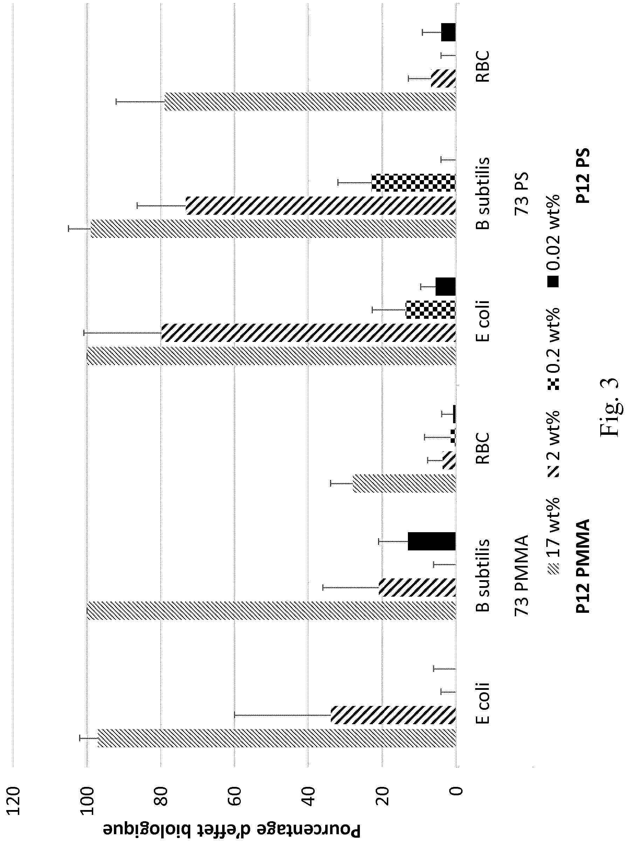

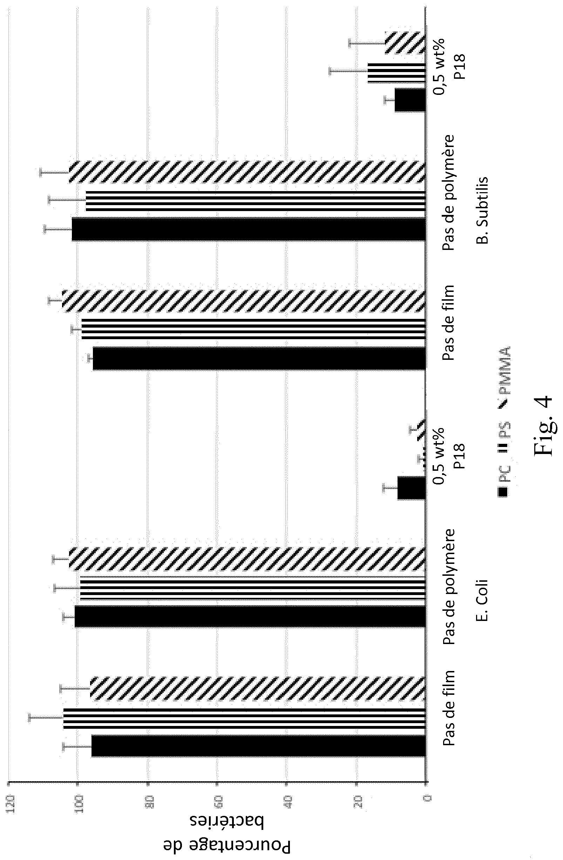

Les Figures 1 à 4 représentent des schémas illustrant l'activité antibactérienne et le caractère hémolytique de matériaux solides selon des exemples de réalisation de la présente description. Le polymère P18 mentionné à la Figure 4, ainsi que le matériau l'incorporant, ne font pas partie de l'objet revendiqué.

### DESCRIPTION DETAILLEE

Dans la description détaillée suivante, des modes de réalisation de la présente description, de nombreux détails spécifiques sont exposés afin de fournir une compréhension plus approfondie de la présente description. Cependant, il apparaîtra à l'homme du métier que la présente description peut être mise en œuvre sans ces détails spécifiques. Dans d'autres cas, des caractéristiques bien connues n'ont pas été décrites en détail pour éviter de compliquer inutilement la description.

Dans ce qui suit, le terme « comprendre » est synonyme de (signifie la même chose que) « inclure », « contenir », et est inclusif ou ouverts et n'exclut pas d'autres éléments non décrits ou représentés. En outre, dans la présente description, les termes « environ » et « à peu près » sont synonymes de (signifie la même chose que) une marge inférieure et/ou supérieure de 10% de la valeur respective.

La présente description concerne un matériau solide organique. Le matériau solide organique comprend au moins un copolymère amphiphile à blocs du type méthacrylique, le copolymère à blocs étant dispersé dans une matrice polymérique, le copolymère à blocs présentant une masse molaire moyenne en nombre *(Mn)* supérieure ou égale à 20 000 g/mol. Le copolymère à blocs comprend au moins un bloc méthacrylique hydrophile comprenant au moins un groupe amine tertiaire et au moins un groupe ion ammonium quaternaire.

Selon un ou plusieurs modes de réalisation, le matériau solide organique possède des propriétés antibactériennes, antimicrobiennes, antiinflammatoires et/ou antifongiques.

Selon un ou plusieurs modes de réalisation, une dispersion améliorée du copolymère à blocs dans la matrice polymérique est obtenue sans pour autant augmenter la toxicité du matériau solide organique ou diminuer l'activité antibactérienne, antimicrobienne, antiinflammatoire et/ou antifongique.

Selon un ou plusieurs modes de réalisation, les matériaux solides organiques sont notamment actifs contre des bactéries du type Gram + et Gram - et pathogènes.

Selon un ou plusieurs modes de réalisation, les matériaux solides organiques ne perdent pas leur activité dans le temps et ne nécessitent pas d'être remplacés en cas d'utilisation prolongée ou détérioration.

Selon un ou plusieurs modes de réalisation, les matériaux solides organiques ne relarguent pas de composés biocides dans l'environnement.

Selon un ou plusieurs modes de réalisation, le matériau solide organique est adapté pour éviter le relargage de molécules et donc la perte d'activité avec le temps, les problèmes d'effets secondaires et de contamination du patient et/ou de l'environnement.

Selon un ou plusieurs modes de réalisation, le matériau solide organique est entièrement antibactérien, et non pas seulement la surface du matériau, atout important en cas de dégradation dudit matériau.

La présente description concerne également un procédé de fabrication d'un matériau solide organique, tel que le matériau solide organique selon le premier aspect, le procédé comprenant la dispersion de 0,02 à 2% d'au moins un copolymère amphiphile à blocs du type méthacrylique dans une matrice polymérique, dans lequel le copolymère à blocs présente une masse molaire moyenne en nombre *(Mn)* supérieure ou égale à 20 000 g/mol, et dans lequel le copolymère à blocs comprend au moins un bloc méthacrylique hydrophile comprenant au moins un groupe amine tertiaire et au moins un groupe ion ammonium quaternaire.

Selon un ou plusieurs modes de réalisation, le procédé selon le deuxième aspect est un procédé simple, versatile, adapté à une large gamme de matrices polymériques (e.g. PS, PMMA, PVC, silicones et composites thermodurcissables) et extrapolable à grande échelle permettant de conférer des propriétés permanentes antibactériennes et antimicrobiennes contre différentes souches de bactéries (Gram + (*B. subtilis*) et Gram - (*E. coli*)) aux matrices polymériques classiques. De plus, ces matériaux solides organiques peuvent présenter des intérêts en tant que matériaux antifongiques, antiviraux et anti-inflammatoires.

Selon un ou plusieurs modes de réalisation, le procédé permet l'élaboration de matériaux solides organiques selon le premier aspect par simple dispersion, dans des matrices polymériques classiques (e.g. PS, PVC, PMMA), de quantités, préférablement faibles (e.g. < 10% en poids), de copolymères amphiphiles à blocs du type méthacrylique, le copolymère pouvant être utilisé comme additif à faible pourcentage en poids.

Selon un ou plusieurs modes de réalisation, les copolymères amphiphiles à blocs du type méthacrylique sont aptes à s'auto-organiser dans différentes matrices leur assurant une dispersion homogène dans le matériau solide organique.

Selon un ou plusieurs modes de réalisation, le procédé de fabrication présente aussi l'avantage d'éviter le relargage de molécules et donc la perte d'activité avec le temps, les problèmes d'effets secondaires et de contamination du patient et/ou de l'environnement.

Selon un ou plusieurs modes de réalisation, le procédé de fabrication assure que le matériau entier soit antibactérien, atout important en cas de dégradation du matériau.

La présente description concerne également des dispositifs comprenant un matériau solide organique selon le premier aspect. En effet, les matériaux solides organiques selon la présente description sont adaptés pour élaborer des dispositifs antibactériens et/ou antimicrobiens, tels que des cathéters (veineux), ne nécessitant plus leur remplacement régulier (environ toutes les 3 semaines) en cas d'utilisation prolongée. Ces dispositifs peuvent aussi présenter des intérêts en tant que matériaux antifongiques, et antiinflammatoires.

La présente description concerne également les copolymères amphiphiles à blocs du type méthacrylique considérés seuls. Spécifiquement, la présente description concerne un copolymère amphiphile à blocs du type méthacrylique présentant une masse molaire moyenne en nombre *(Mn)* supérieure ou égale à 20 000 g/mol, par exemple pour application antibactérienne et/ou antimicrobienne, anti-inflammatoire et/ou antifongique, le copolymère à blocs comprenant au moins un bloc méthacrylique hydrophile comprenant au moins un groupe amine tertiaire et au moins un groupe ion ammonium quaternaire.

Des exemples de copolymères amphiphiles à blocs du type méthacrylique, de matériaux solides organiques et de procédés de fabrication selon les aspects précités sont décrits ci-dessous.

### Synthèse et caractérisation de copolymères dibloc amphiphiles PBMA-b-PDMAEMA

Une série de copolymères dibloc amphiphiles PBMA-*b*-PDMAEMA ont été préparés par polymérisation radicalaire contrôlée par des nitroxydes (NMP) en utilisant l'alcoxyamine BlocBuilder MA comme amorceur et en ajoutant un comonomère pour le contrôle des masses molaires et la distribution des masses molaires. Dans ces exemples, 10% en moles de styrène (S) ou d'acrylonitrile (ACN) ont été utilisés. Les copolymères dibloc ont la structure suivante : 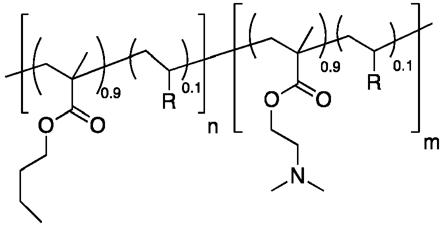 dans laquelle n et m sont le degré de polymérisation de chaque bloc, et R est un styrène ou un acrylonitrile. Dans ces exemples, le pourcentage de groupe hydrophobe et le poids moléculaire du copolymère dibloc amphiphiles ont été variés.

La synthèse de (PBMA₇₀₀₀-*b*-PDMAEMA₄₅₈₀₀) (**P8**) est présentée ici comme exemple de procédé de fabrication selon le second aspect. Cette synthèse est applicable à tous les copolymères à blocs selon la présente description. On chauffe à 90° C sous atmosphère d'azote du BMA (monomère hydrophobe, 50 g, 0,35 mole), du styrène (3,66 g, 0,035 mole), du BlocBuilder MA (1,27 g, 3,33 mmoles) et du SG1 (nitroxyde *N*-(2-methylpropyl)-*N*-(1-diethylphosphono-2,2-dimethylpropyl)-*N*-oxyl), 100 mg, 0,33 mmole). Des échantillons sont prélevés périodiquement pour surveiller la conversion et la masse molaire. La réaction est arrêtée lorsque la conversion atteint 45% (e.g. 2 heures). La conversion est déterminée par ¹H RMN (spectromètre Bruker 300 MHz, CDCl₃) en comparant les pics des -CH₂- proche de la fonction ester du monomère (δ = 4,11 ppm) et du polymère (δ = 3,91 ppm). Le PBMA est ensuite récupéré par précipitation dans un mélange de MeOH/H₂O à froid (4/1 en volume) et analysé par SEC/DMF (chromatographie d'exclusion stérique PL120 (Polymer Laboratories, England) avec comme éluant du DMF) pour obtenir les valeurs de masse molaire moyenne en nombre (*Mn*) et de dispersité (Ð ; *Mw*/*Mn*) (*Mn* = 7000 g.mol⁻¹, D = 1.41). Après séchage pendant 24h sous vide, le PBMA est utilisé pour amorcer la polymérisation du monomère hydrophile (DMAEMA) et obtenir le copolymère dibloc PBMA-*b*-PDMAEMA correspondant. Pour ce faire, on chauffe à 90° C sous atmosphère d'azote du PBMA (1 g), du styrène (662 mg, 6,36 mmoles), du DMAEMA (10 g, 63,6 mmoles), du 1,4-dioxane (5 ml) et du SG1 (4 mg, 0,0125 mmole). Des échantillons sont prélevés périodiquement pour surveiller la conversion par ¹H RMN (spectromètre Bruker CDCl₃, 400 MHz) et la valeur du *Mn* est déterminée par SEC/DMF. La réaction est arrêtée lorsque la conversion est proche de 50%. Le copolymère dibloc PBMA-*b*-PDMAEMA final est ensuite isolé par précipitation dans du pentane à froid. Le copolymère obtenu P8 est analysé par ¹H RMN et SEC/DMF pour obtenir une composition ayant un F_{DMAEMA} = 0,60 (ratio molaire de DMAEMA dans le copolymère final), une *Mn =* 45800 g.mol⁻¹ et un D = 1.37.

### Synthèse et caractérisation de copolymères diblocs amphiphiles PMMA-b-PDMAEMA

Une série de copolymères dibloc amphiphiles PMMA-*b*-PDMAEMA ont été préparés par polymérisation radicalaire contrôlée par des nitroxydes (NMP) en utilisant l'alcoxyamine BlocBuilder MA comme amorceur et en ajoutant un comonomère pour le contrôle des masses molaires et la distribution des masses molaires. Dans ces exemples, 10% en moles de styrène (S) ou d'acrylonitrile (ACN) ont été utilisés. Les copolymères dibloc ont la structure suivante : 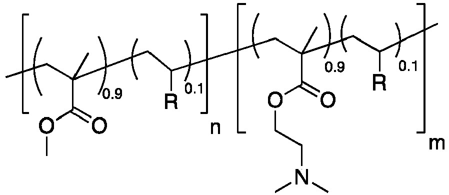 dans laquelle n et m sont le degré de polymérisation de chaque bloc, et R est un styrène ou un acrylonitrile.

La synthèse de (PMMA₉₃₀₀-*b*-PDMAEMA₄₀₀₀₀) (**P1**) est présentée ici comme exemple de procédé de fabrication selon le second aspect. Cette synthèse est applicable à tous les copolymères à blocs selon la présente description. On chauffe à 90° C sous atmosphère d'azote du MMA (méthyl méthacrylate, 50 g, 0,50 mole), du styrène (5,2 g, 0,05 mole), du BlocBuilder MA (954 mg, 2,5 mmoles) et du SG1 (74 mg, 0,25 mmole). Des échantillons sont prélevés périodiquement pour surveiller la conversion et la masse molaire. La réaction est arrêtée lorsque la conversion atteint 45% (e.g. 2 heures). La conversion est déterminée par ¹H RMN (spectromètre Bruker 300 MHz, CDCl₃) en comparant les pics des -CH₃- proche de la fonction ester du monomère (δ = 4,11 ppm) et du polymère (δ = 3,91 ppm). Le PMMA est ensuite récupéré par précipitation dans un mélange de MeOH/H₂O à froid (4/1 en volume) et analysé par SEC/DMF pour obtenir les valeurs de masse molaire moyenne en nombre *(Mn)* et de dispersité (D) (*Mn* = 9300 g.mol⁻¹, D = 1.30). Après séchage pendant 24h sous vide, le PMMA est utilisé pour amorcer la polymérisation du DMAEMA et obtenir le copolymère dibloc PMMA-*b*-PDMAEMA correspondant. Pour ce faire, on chauffe à 90° C sous atmosphère d'azote du PMMA (1 g), du styrène (662 mg, 6,37 mmoles), du DMAEMA (10 g, 63,6 mmoles), du 1,4-dioxane (5 ml) et du SG1 (4 mg, 0,0125 mmole). Des échantillons sont prélevés périodiquement pour surveiller la conversion par ¹H RMN (spectromètre Bruker CDCl₃, 400 MHz) et la valeur du *Mn* est déterminée par SEC/DMF. La réaction est arrêtée lorsque la conversion est proche de 50%. Le copolymère dibloc PMMA-*b*-PDMAEMA final est ensuite isolé par précipitation dans du pentane à froid. Le copolymère obtenu **P1** est analysé par ¹H RMN et SEC/DMF pour obtenir une composition ayant un F_{DMAEMA} = 0,74, un *Mn* = 40000 g.mol⁻¹ et un D = 1.46.

### Synthèse et caractérisation de copolymères diblocs amphiphiles PMMA-b-PDEAEMA

Une série de copolymères dibloc amphiphiles PMMA-*b*-PDEAEMA ont été préparés par polymérisation radicalaire contrôlée par des nitroxydes (NMP) en utilisant l'alcoxyamine BlocBuilder MA comme amorceur et en ajoutant un comonomère pour le contrôle des masses molaires et la distribution des masses molaires. Dans ces exemples, 10% en moles de styrène (S) ou d'acrylonitrile (ACN) ont été utilisés. Les copolymères dibloc ont la structure suivante : 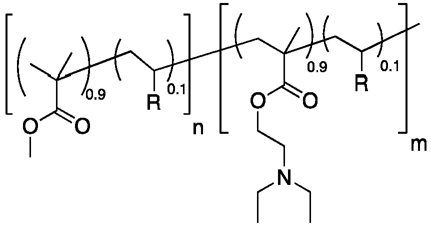 dans laquelle n et m sont le degré de polymérisation de chaque bloc, et R est un styrène ou un acrylonitrile.

La synthèse de (PMMA₉₃₀₀-*b*-PDEAEMA₂₅₅₀₀) (**P2**) est présentée ici comme exemple de procédé de fabrication selon le second aspect. Cette synthèse est applicable à tous les copolymères à blocs selon la présente description. On chauffe à 90° C sous atmosphère d'azote du MMA (méthyl méthacrylate, 50 g, 0,50 mole), du styrène (5,2 g, 0,05 mole), du BlocBuilder MA (954 mg, 2,5 mmoles) et du SG1 (74 mg, 0,25 mmole). Des échantillons sont prélevés périodiquement pour surveiller la conversion et la masse molaire. La réaction est arrêtée lorsque la conversion atteint 45% (e.g. 2 heures). La conversion est déterminée par ¹H RMN (spectromètre Bruker 300 MHz, CDCl₃) en comparant les pics des -CH₃- proche de la fonction ester du monomère (δ = 4,11 ppm) et du polymère (δ = 3,91 ppm). Le PMMA est ensuite récupéré par précipitation dans un mélange de MeOH/H₂O à froid (4/1 en volume) et analysé par SEC/DMF pour obtenir les valeurs de masse molaire moyenne en nombre *(Mn)* et de dispersité (Ð) (*Mn* = 9300 g.mol⁻¹, D = 1.30). Après séchage pendant 24h sous vide, le PMMA est utilisé pour amorcer la polymérisation du monomère hydrophile de méthacrylate de 2-(diéthylamino)éthyle (DEAEMA) et obtenir le copolymère dibloc PMMA-*b*-PDEAEMA correspondant. Pour ce faire, on chauffe à 90° C sous atmosphère d'azote du PMMA (1,3 g), du styrène (562 mg, 5,4 mmoles), du DEAEMA (10 g, 54 mmoles), du 1,4-dioxane (5 ml) et du SG1 (3 mg, 0,01 mmole). Des échantillons sont prélevés périodiquement pour surveiller la conversion par ¹H RMN (spectromètre Bruker CDCl₃, 400 MHz) et la valeur du *Mn* est déterminée par SEC/DMF. La réaction est arrêtée lorsque la conversion est proche de 50%. Le copolymère dibloc PMMA-*b*-PDEAEMA final est ensuite isolé par précipitation dans du pentane à froid. Le copolymère obtenu **P2** est analysé par ¹H RMN et SEC/DMF pour obtenir une composition ayant un F_{DMAEMA} = 0,58, un *Mn* = 25500 g.mol⁻¹ et un D = 1.30.

### Synthèse et caractérisation de copolymères dibloc amphiphiles PBMA-b-PDEAEMA

Une série de copolymères dibloc amphiphiles PBMA-*b*-PDEAEMA ont été préparés par polymérisation radicalaire contrôlée par des nitroxydes (NMP) en utilisant l'alcoxyamine BlocBuilder MA comme amorceur et en ajoutant un comonomère pour le contrôle des masses molaires et la distribution des masses molaires. Dans ces exemples, 10% en moles de styrène (S) ou d'acrylonitrile (ACN) ont été utilisés. Les copolymères dibloc ont la structure suivante : 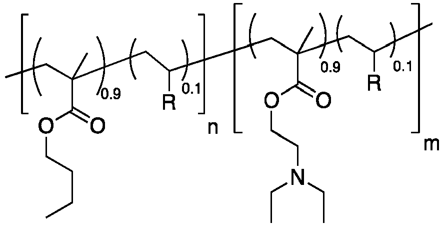 dans laquelle n et m sont le degré de polymérisation de chaque bloc, et R est un styrène ou un acrylonitrile. Dans ces exemples, le pourcentage de groupe hydrophobe et le poids moléculaire du copolymère dibloc amphiphiles ont été variés.

La synthèse de (PBMA₉₃₀₀-*b*-PDEAEMA₁₈₀₀₀) (**P4**) est présentée ici comme exemple de procédé de fabrication selon le second aspect. Cette synthèse est applicable à tous les copolymères à blocs selon la présente description. On chauffe à 90° C sous atmosphère d'azote du BMA (monomère hydrophobe, 50 g, 0,35 mole), du styrène (3,66 g, 0,035 mole), du BlocBuilder MA (952 g, 2,49 mmoles) et du SG1 (73 mg, 0,25 mmole). Des échantillons sont prélevés périodiquement pour surveiller la conversion et la masse molaire. La réaction est arrêtée lorsque la conversion atteint 45% (e.g. 2 heures). La conversion est déterminée par ¹H RMN (spectromètre Bruker 300 MHz, CDCl₃) en comparant les pics des -CH₂- proche de la fonction ester du monomère (δ = 4,11 ppm) et du polymère (δ = 3,91 ppm). Le PBMA est ensuite récupéré par précipitation dans un mélange de MeOH/H₂O à froid (4/1 en volume) et analysé par SEC/DMF pour obtenir les valeurs de masse molaire moyenne en nombre *(Mn)* et de dispersité (Ð ; *Mw*/*Mn*) (*Mn* = 9300 g.mol⁻¹, D = 1.24). Après séchage pendant 24h sous vide, le PBMA est utilisé pour amorcer la polymérisation du monomère hydrophile (DMAEMA) et obtenir le copolymère dibloc PBMA-*b*-PDEAEMA correspondant. Pour ce faire, on chauffe à 90° C sous atmosphère d'azote du PBMA (1,5 g), du styrène (562 mg, 5,4 mmoles), du DEAEMA (10 g, 54 mmoles), du 1,4-dioxane (5 ml) et du SG1 (4 mg, 0,0125 mmole). Des échantillons sont prélevés périodiquement pour surveiller la conversion par ¹H RMN (spectromètre Bruker CDCl₃, 400 MHz) et la valeur du *Mn* est déterminée par SEC/DMF. La réaction est arrêtée lorsque la conversion est proche de 50%. Le copolymère dibloc PBMA-*b*-PDEAEMA final est ensuite isolé par précipitation dans du pentane à froid. Le copolymère obtenu **P4** est analysé par ¹H RMN et SEC/DMF pour obtenir une composition ayant un F_{DMAEMA} = 0,31, un *Mn =* 18000 g.mol⁻¹ et un D = 1.52.

### Ouaternisation des copolymères à blocs

La quaternisation des exemples de copolymères dibloc a été effectuée par modification de la fonction amine de DMAEMA ou de DEAEMA avec de l'iodure de méthyle (MeI). Des rendements différents de quaternisation ont été ciblés, tel que 0% (c'est-à-dire pas de quaternisation, ne faisant donc pas partie de l'objet revendiqué), 2%, et 100% (c'est-à-dire la quaternisation de toutes les fonctions amines, ne faisant donc pas partie de l'objet revendiqué). Le copolymère dibloc est solubilisé dans un solvant (e.g. 1 g de copolymère dans 10 ml de DMF) et du MeI est ajouté à la solution, par exemple lentement à température ambiante. Après 24h sous agitation, le copolymère quaternisé est dialysé contre de l'eau ultrapure (e.g. eau Milli-Q^{®}) pendant 3 jours puis lyophilisé avant analyse par ¹H RMN.

**Table 1 : copolymère amphiphile à blocs du type méthacrylique P1 à P18 (avec 10% en moles de styrène ou acrylonitrile par monomère méthacrylique)**

| # | copolymère à blocs | S ou ACN | % du bloc hydrophile | % quat. Mel | *Mn* (g/mol) | *Mn* du bloc Hydrophobe (g/mol) | Ð |
|---|---|---|---|---|---|---|---|
| **P1** | PMMA-*b-*PDMAEMA | S | 74% | 2 mol% | **40 000** | **9 300** | 1.46 |
| **P2** | PM MA-*b-*PDEAEMA | S | 58% | 2 mol% | **25 500** | **9 300** | 1.30 |
| **P3** | PBMA-*b-*PDMAEMA | S | 49% | 2 mol% | **35 200** | **9 300** | 1.28 |
| **P4** | PBMA-*b-*PDEAEMA | S | 31% | 2 mol% | **18 000** | **9 300** | 1.52 |
| **P5** | PBMA-*b-*PDMAEMA | S | 46% | 2 mol% | **61 600** | **20 200** | 1.60 |
| **P6** | PBMA-*b-*PDMAEMA | S | 1-9% | 2 mol% | **28 700** | **20 200** | 1.24 |
| **P7** | PBMA-*b*- | S | 38% | 2 mol% | **17 700** | **7 000** | 1.38 |
| | PDMAEMA | | | | | | |
| **P8** | PBMA-*b-*PDMAEMA | S | 60% | 2 mol% | **45 800** | **7 000** | 1.37 |
| **P9** | PBMA-*b-*PDMAEMA | S | 40% | 2 mol% | **22 900** | **9 300** | 1.24 |
| **P10** | PBMA-*b-*PDMAEMA | S | 37% | 2 mol% | **22 600** | **9 300** | 1.27 |
| **P11** | PBMA-*b-*DMAEMA | S | 42% | 2 mol% | **22 100** | **7 000** | 1.26 |
| **P12** | PBMA-*b-*PDMAEMA | S | 62% | 2 mol% | **52 800** | **7 000** | 1.26 |
| **P13** | PBMA-*b-*PDMAEMA | ACN | 35% | 2 mol% | **22 400** | **12 800** | 1.32 |
| **P14 *** | PBMA-*b-*PDMAEMA | S | 73% | 0 mol% | **31 300** | **6 400** | 1.54 |
| **P15 *** | PBMA-*b-*PDMAEMA | ACN | 64% | 0 mol% | **20 500** | **9 300** | 1.34 |
| **P16** * | PBMA-*b-*PDMAEMA | S | 73% | 100 mol% | **31 300** | **6 400** | 1.54 |
| **P17 *** | PBMA-*b-*PDMAEMA | ACN | 64% | 100 mol% | **20 500** | **9 300** | 1.34 |
| **P18 *** | PBMA-*b-*PDMAEMA | ACN | 72% | 100 mol% | **20500** | **9 000** | 1.45 |

| | | | | | | | |
|---|---|---|---|---|---|---|---|
| ** ne faisant pas partie de l'objet revendiqué* | | | | | | | |

### Activités antimicrobiennes des copolymères amphiphile à blocs du type méthacrylique en solution

L'activité antimicrobienne des copolymères P1-P18 a été testée. Diverses souches Gram négatives ont été utilisées : Escherichia coli (ATCC 8739), Pseudomonas aeruginosa (ATCC CRM-9027) et P aeruginosa FQR (Fluoroquinone résistante) (CIP 107398). Des souches Gram positives ont également été utilisées : Staphylococcus aureus (ATCC CRM-6538P) et S. aureus meticilline résistant (MRSA USA300) (ATCC BAA-1717 USA 300 CA-MRSA). L'activité antimicrobienne des copolymères à blocs a été évaluée par la détermination de la concentration inhibitrice minimale (CIM). La CIM a été déterminée en utilisant des dilutions en série par deux d'agents antimicrobiens dans des milieux liquides bactériens tels que décrits par le Comité national des normes cliniques de laboratoire (NCCLS) (NCCLS, 1997). En bref, des colonies individuelles des différentes souches bactériennes cultivées sur des plaques d'agar Luria-Bertani (LB) sont utilisées pour inoculer du milieu de culture bactérien liquide (LB ou Mueller-Hinton (MH)). Les tubes sont incubés pendant une nuit à 37° C sous agitation (200 tr/min). Le lendemain, les suspensions bactériennes (densité optique OD> 1,0) sont diluées 1/100 dans 3 ml de milieu MH ou LB frais et incubées à 37° C, 200 tr/min jusqu'à ce que les bactéries atteignent la phase logarithmique de croissance (OD environ 0,6). Les bactéries sont ensuite diluées dans du milieu MH ou LB pour atteindre une densité bactérienne autour de 10^{E5} bactéries/ml. 135 µl de suspension bactérienne sont ensuite ajoutés dans chacun des 96 puits d'une microplaque de polypropylène stériles (Greiner BioOne) contenant déjà 15 µl d'antimicrobien dilué en série (1:2 dans l'eau distillée). Les plaques sont incubées à 37° C pendant 18-24 h avant la mesure de l'OD₆₀₀ₙₘ en utilisant un lecteur de microplaques. La CIM est définie comme la plus faible concentration d'antimicrobien qui inhibe la croissance visible de l'organisme (99 % d'inhibition). Les résultats sont décrits aux tables 2 et 3, NS étant pour non soluble.

| **Table 2** | **Copolymère à blocs** | **CIM (µM) en milieu LB** | | | |
|---|---|---|---|---|---|
| | | **E coli** | **B subtilis** | **S aureus** | **P aeruginosa** |
| **P1** | PMMA-*b*-PDMAEMA | 2,6 | 1,3 | > 21 | 2,6 |
| **P2** | PMMA-*b*-PDEAEMA | 9,35 | 9,35 | > 37 | 9,35 |
| **P3** | PBMA-*b*-PDMAEMA | 5,75 | 2,88 | > 23 | 2,88 |
| **P4** | PBMA-*b*-PDEAEMA | 10 | 5 | > 40 | 10,00 |
| **P5** | PBMA-*b*-PDMAEMA | 4,77 | 2,37 | > 38 | 5 |
| **P6** | PBMA-*b*-PDMAEMA | NS | NS | NS | NS |
| **P7** | PBMA-*b*-PDMAEMA | 5 | >20 | | |
| **P8** | PBMA-*b*-PDMAEMA | 0,98 | 1 | > 15,8 | 1 |
| **P9** | PBMA-*b*-PDMAEMA | 5 | 3 | | |
| **P10** | PBMA-*b*-PDMAEMA | 3 | 1 | | |
| **P11** | PBMA-*b*-DMAEMA | 20 | 20 | | |
| **P12** | PBMA-*b*-PDMAEMA | 1 | 1 | | |
| **P13** | PBMA-*b*-PDMAEMA | 20 | 10 | | |
| | | | | | |
| **P14*** | PBMA-*b*-PDMAEMA | 2,5 | | | |
| **P15*** | PBMA-*b*-PDMAEMA | 10 | | | |
| **P16*** | PBMA-*b*-PDMAEMA | >20 | | | |
| **P17*** | PBMA-*b*-PDMAEMA | 5 | | | |

| | | | | | |
|---|---|---|---|---|---|
| ** ne faisant pas partie de l'objet revendiqué* | | | | | |

| **Table 3** | **Copolymère à blocs** | **CIM (µM) en milieu MH** | | | | | |
|---|---|---|---|---|---|---|---|
| | | **E coli** | **B subtilis** | **S aureus** | **Pseudomonas FQR** | **MRSA** | **E faecalis VER** |
| **P1** | PMMA-*b*-PDMAEMA | 5,250 | 0,660 | 21,300 | 2,6 | 2,6 | >21,3 |
| **P2** | PMMA-*b*-PDEAEMA | > 37 | >37 | > 37 | 9,35 | >37 | >37,4 |
| **P3** | PBMA-*b*-PDMAEMA | 5,750 | 0,360 | 23,100 | 1,4 | 2,8 | >23,1 |
| **P4** | PBMA-*b*-PDEAEMA | > 40 | 20,000 | > 40 | 10 | 5 | >40,2 |
| **P5** | PBMA-*b*-PDMAEMA | 9,550 | 1,180 | 19,100 | 2,4 | 4,75 | 38,2 |
| **P6** | PBMA-*b*-PDMAEMA | NS | NS | NS | NS | NS | NS |
| **P7** | PBMA-*b*-PDMAEMA | >20 | >20 | | 6,5 | >20 | >26,1 |
| **P8** | PBMA-*b*-PDMAEMA | 1,975 | 0,490 | 7,900 | 0,98 | 0,98 | 15,8 |
| **P9** | PBMA-*b*-PDMAEMA | 1,250 | 0,313 | | 2,5 | 5 | 20 |
| **P10** | PBMA-*b*-PDMAEMA | 1,250 | 0,625 | | 2,5 | 5 | >20 |
| **P11** | PBMA-*b*-DMAEMA | >20 | 0,625 | | 20 | 1,25 | 1,25 |
| **P12** | PBMA-*b*-PDMAEMA | 0,625 | 0,150 | | 2,5 | 1,25 | 20 |
| | | | | | | | |
| **P13** | PBMA-*b*-PDMAEMA | 20,000 | 10,000 | | 20 | 1,25 | 2,5 |
| **P14*** | PBMA-*b*-PDMAEMA | 0,6 | 0,3 | | 0,3 | 0,6 | |
| **P15*** | PBMA-*b*-PDMAEMA | 0,6 | 0,3 | | 2.5 | 1,25 | |
| **P16*** | PBMA-*b*-PDMAEMA | 0,6 | 0,6 | | 1,25 | 0,15 | |
| **P17*** | PBMA-*b*-PDMAEMA | 0,6 | 0,15 | | 0,6 | 0,07 | |
| P18* | PBMA-*b*-PDMAEMA | 2,5 | 0,31 | | 5,0 | 0,31 | |

| | | | | | | | |
|---|---|---|---|---|---|---|---|
| ** ne faisant pas partie de l'objet revendiqué* | | | | | | | |

### Caractères hémolytiques des copolymères amphiphiles à blocs du type méthacrylique en solution

Le caractère hémolytique des copolymères **P1-P17** a été testé en solution. Spécifiquement, l'activité hémolytique a été déterminée par la perte d'hémoglobine à partir d'érythrocytes humains (obtenus auprès de Divbioscience, NL). Les érythrocytes humains ont été lavés 3 fois avec une solution saline tamponnée au phosphate (PBS) et concentrés par centrifugation à 800 g pendant 5 min. Les érythrocytes humains ont ensuite été remis en suspension dans du PBS à une concentration finale de 8%. 135 µl d'érythrocytes humains ont ensuite été ajoutés dans chacun des 96 puits d'une microplaque stérile contenant 15 µl d'antimicrobiens dilués (1:2 dans de l'eau distillée). Après 1 h à 37° C, la microplaque a été centrifugée à 800 g pendant 5 min. 100 µl ont ensuite été soigneusement collectés et transférés dans une nouvelle microplaque à 96 puits et la DO a été mesurée à 405 nm. L'hémolyse provoquée par les copolymères a été exprimée en pourcentage d'hémolyse, le Triton-X100 à 0,1% (v:v) étant utilisé comme témoin positif et donnant 100% d'hémolyse. Les résultats sont décrits à la Table 4.

*Tables 2-4 : activités antibactériennes et caractères hémolytiques des copolymères diblocs en solution* (HMC représente la concentration causant 100% d'hémolyse et HC50 la concentration causant 50% d'hémolyse ; hRBC et sRBC sont pour « human red blood cell » et « sheep red blood cell », respectivement).

**Table 4**

| **Copolymère à blocs** | | **HMC (µM)** | | **HC50 (µM)** | |
|---|---|---|---|---|---|
| | | **hRBC** | **sRBC** | **hRBC** | **sRBC** |
| **P1** | PMMA-*b*-PDMAEMA | 10,6 | > 21,3 | 2,6-5,3 | > 21,3 |
| **P2** | PMMA-*b*-PDEAEMA | <0,36 | <0,36 | <0,36 | <0,36 |
| **P3** | PBMA-*b*-PDMAEMA | 5,75 | >23,1 | 1,4-2,8 | >23,1 |
| **P4** | PBMA-*b*-PDEAEMA | <0,39 | <0,39 | <0,39 | <0,39 |
| **P5** | PBMA-*b*-PDMAEMA | 5 | >38 | 0,59-1,19 | >38 |
| **P6** | PBMA-*b*-PDMAEMA | NS | NS | NS | NS |
| **P7** | PBMA-*b*-PDMAEMA | <0,3 | <0,3 | <0,3 | <0,3 |
| **P8** | PBMA-*b*-PDMAEMA | 1 | 2 | 0,24-0,49 | 0,47-0,9 |
| **P9** | PBMA-*b*-PDMAEMA | <0,3 | <0,3 | <0,3 | <0,3 |
| **P10** | PBMA-*b*-PDMAEMA | <0,3 | <0,3 | <0,3 | <0,3 |
| **P11** | PBMA-*b*-DMAEMA | >20 | >20 | 1,25 | 20,00 |
| **P12** | PBMA-*b*-PDMAEMA | <0,3 | 20,00 | <0,3 | 1,25 |
| **P13** | PBMA-*b*-PDMAEMA | >20 | >20 | >20 | >20 |
| **P14*** | PBMA-*b*-PDMAEMA | | | 0,039 | |
| **P15*** | PBMA-*b*-PDMAEMA | | | >20 | |
| **P16*** | PBMA-*b*-PDMAEMA | | | >20 | |
| **P17*** | PBMA-*b*-PDMAEMA | | | >20 | |

| | | | | | |
|---|---|---|---|---|---|
| ** ne faisant pas partie de l'objet revendiqué* | | | | | |

### Activité antifongique d'un copolymère exemplaire amphiphile à blocs du type méthacrylique en solution

Des tests antifongiques ont été réalisés sur deux souches: Aspergillus niger et Candida albicans. Aspergillus niger a été ensemencé sur des boites d'agar PDA et incubé à 35°C jusqu'à sporulation (72h). Une fois la sporulation atteinte, 1 ml de tampon salin 0.85% contenant du tween 20 est ajouté afin de faciliter la dissociation du mycélium. Une fois filtrées sur gaze stérile, les spores sont comptées et diluées dans du milieu PD de telle sorte à atteindre une concentration proche de 5 X10E4 CFU/ml. Les spores sont ensuite ajoutées aux composés dilués dans des boîtes de 96 puits et incubées 48 h à 35°C. Candida albicans a été ensemencé sur boite d'agar LB et incubée à 35°C sur la nuit. Le jour suivant cinq colonies sont collectées de la boite et ajoutées à 5 ml de tampon salin. Après lecture de la densité optique à 600nm (1 unité équivalent à 10⁷ CFU/ml), les levures sont diluées dans du milieu RPMI + MOPS afin d'atteindre une concentration de 10³ CFU /ml. Les levures sont ajoutées alors dans une plaque de 96 puits en présence de concentrations croissantes de composés et sont incubées 48 h à 35°C. Les CMI sont déterminées pour Aspergillus niger et Candida albicans comme étant la plus faible concentration en composé entrainant l'absence de croissance fongique évaluée par mesure de l'absorbance des puits à 600nm. Les tests montrent que le composé P14 présente une CMI de 40 µM sur les champignons.

### Activité anti-inflammatoire des copolymères amphiphiles à blocs du type méthacrylique en solution

L'activité anti-inflammatoire des composés a été testée sur monocytes humains (cellules THP-1) stimulés par du lipopolysaccharide (LPS) (extrait d'E coli et utilisé à la concentration finale de 100 ng/ml). Des cellules THP-1 sont ensemencées en plaques de 96 puits à raison de 10⁴ cellules par puits. Les cellules sont ensuite traitées avec du LPS (à 100 ng/ml) en présence ou non de composés en concentration croissante. Après 6 h d'incubation, le surnageant de culture est collecté avant mesure de la concentration de TNF-alpha, une cytokine témoin de l'inflammation et induite par le LPS. La polymyxine B, un agent inhibant l'effet du LPS, a été utilisée en contrôle positif. La viabilité cellulaire des cellules THP-1 a également mesurée par le test de bleu d'Alamar. Les concentrations inhibitrices 50 (IC50) causant 50% d'inhibition de la production de TNF-alpha ou de la viabilité cellulaire ont été déterminées visuellement (Table 5). Le « safety factor » a été déterminé en divisant l'IC50 sur la production de TNF-alpha par l'IC50 sur la viabilité.
Les résultats sont décrits à la Table 5.

| **Table 5** | **Copolymère à blocs** | **IC50 inflammation (µM)** | **HC50 viabilité (µM)** | **Safety Factor** |
|---|---|---|---|---|
| **P14*** | PBMA-*b*-PDMAEMA | 0,019 | 20 | 1000 |
| **P15*** | PBMA-*b*-PDMAEMA | 0,15 | >20 | >100 |
| **P16*** | PBMA-*b*-PDMAEMA | 0,039 | >20 | >500 |
| **P17*** | PBMA-*b*-PDMAEMA | 0,078 | >20 | >250 |

| | | | | |
|---|---|---|---|---|
| ** ne faisant pas partie de l'objet revendiqué* | | | | |

### Procédé de fabrication du matériau solide organique par évaporation de solvant

A l'issu de tests biologiques en solution des copolymères préparés, différents matériaux solides organiques par évaporation de solvant à base de PS et PMMA contenant différentes quantités de copolymères à base de butyl méthacrylate (BMA) et de N,N-diméthyl-amino éthyl méthacrylate (DMAEMA) (quaternisé ou pas) ont été préparés et testés.

Les matrices polymériques à base de PS et PMMA ont été préparées par la même méthode d'évaporation de solvant. Par exemple, dans le cas du PS, une solution stock de 20% en poids de PS dans du THF est préparée. 226 mg de copolymère PX (*i.e.,* P1-P17) sont dissouts dans 5 ml de la solution de PS à 20% dans le THF pour donner une solution de copolymère/PS à 17% en poids. Le même mode opératoire est utilisé pour préparer une solution de copolymère à 2% en poids/PS en utilisant 22,6 mg de copolymère et 5 ml de la solution de PS 20%/THF. 500 µl de la solution de copolymère à 2% en poids/PS sont ensuite dilués avec 4,5 ml de la solution de PS20%/THF pour donner une solution de copolymère/PS à 0,2% en poids. Le même procédé est utilisé pour préparer des solutions de copolymère/PS à 0,02% en poids. Après 1 heure d'agitation à température ambiante, 150 µl de chaque solution sont versés dans chacun des 96 puits d'une microplaque de polypropylène stériles (Greiner BioOne). Une ligne de la microplaque est consacrée à chacune des différentes concentrations de copolymère et une ligne est remplie avec seulement une solution PS20%/THF comme essai à blanc. Après 2 jours d'évaporation du THF à température ambiante, les films de copolymère/PS obtenus sont ensuite testés en terme d'hémolyse et en terme d'activité antimicrobienne contre *E. coli* et *B. subtilis.*

### Activités antimicrobiennes d'une série matériaux solides organiques préparés par évaporation de solvant

L'activité antimicrobienne de matériaux solides organiques préparés par évaporation de solvant contenant des copolymères dibloc **P5, P8** et **P12** ont été testés. L'activité antimicrobienne a été évaluée selon une procédure adaptée ISO 22196. La matrice polymérique (PS ou PMMA) contenant le copolymère dibloc est introduite dans des microplaques à 96 puits de polypropylène stériles (Greiner BioOne). Des suspensions bactériennes sont préparées comme réalisé pour les essais CIM, à la différence que la suspension bactérienne en phase logarithmique de croissance (OD600 nm environ 0,6) est centrifugée à 6 000 tr/min pendant 10 min à 4° C. Les culots bactériens sont lavés avec 10 ml de solution saline stérile tamponnée au phosphate (PBS) et centrifugés (6 000 tr/min, 10 min, 4° C). Les bactéries sont finalement diluées dans du PBS stérile pour atteindre une densité bactérienne autour de 10^{E5} bactéries/ml. 10 µl de ces suspensions (correspondant à 1 000 bactéries) sont ajoutés sur la surface de copolymères polymérisés. Des témoins négatifs sont obtenus en ajoutant la suspension bactérienne de 10 µl directement sur la surface du polymère témoin sans addition de copolymère à blocs (PMMA ou PS). Après 60 minutes d'incubation à température ambiante, les bactéries sont collectées en ajoutant 90 µl de PBS stérile sur la surface et en répétant le pipetage vers le haut et vers le bas. Les bactéries sont ensuite diluées en série (dilution 1 sur 10) dans du PBS stérile avant d'étaler 10 µl de la suspension bactérienne sur des plaques de gélose LB. Après une nuit d'incubation à 37° C, des plaques sont observées et les colonies sont comptées. Le nombre de bactéries viables a été exprimé en fonction du nombre d'unités de formation de colonies (CFU) observées. Les expériences ont été effectuées en trois exemplaires indépendants (n = 3). Les résultats des tests antimicrobiens effectués sur les matériaux solides organiques sont présentés dans les Figures 1 à 3.

### Caractères hémolytiques d'une série matériaux solides organiques

Le caractère hémolytique de matériaux solides organiques contenant des copolymères dibloc **P5, P8** et **P12** ont été testés. 10 µl de suspension d'érythrocytes humains (8% dans PBS) sont ajoutés dans chacun de 96 puits d'une microplaque contenant matériaux solides organiques (PS ou PMMA avec un copolymère dibloc). Après 60 minutes d'incubation à température ambiante, 90 µl de PBS sont ajoutés et la microplaque est centrifugée à 800 g pendant 5 min. 50 µl sont ensuite soigneusement collectés des puits et transférés dans une nouvelle microplaque à 96 puits et la DO est mesurée à 405 nm. L'hémolyse provoquée par les copolymères est exprimée en pourcentage d'hémolyse, le Triton-X100 étant utilisé comme témoin positif. Les résultats des tests hémolytiques effectués sur les matériaux solides organiques sont présentés dans les Figures 1 à 3.

Comme montré sur les Figures 1 à 3, les tests effectués sur *E. coli* et *B. subtilis* montrent que les matériaux solides organiques préparés sont actifs jusqu'à 2 wt% et ce, sans caractère hémolytique (tests sur globules rouges humains, RBC).

Bien que des modes de réalisation de la présente description mentionnés ci-dessus soient décrits en détail, il est entendu que d'autres modes de réalisation peuvent être envisagés. Ainsi, par exemple, des matrices polymériques autres que du PMMA ou PS peuvent être envisagées pour obtenir un matériau solide organique selon le premier aspect ; des méthacrylates autres que du DMAEMA ou DEAEMA peuvent être envisagés pour obtenir un matériau solide organique selon le premier aspect ; des copolymères à base de composés autres que du MMA, BMA, S et/ou ACN peuvent être envisagés pour obtenir un matériau solide organique selon le premier aspect.

### Procédé de fabrication du matériau solide organique par extrusion

Différents matériaux solides organiques à base de PS, PMMA et PC (polycarbonate (CALIBRE 201-15 from TRINSEO)) contenant différentes quantités de copolymères à base de butyl méthacrylate (BMA) et de *N*,*N*-diméthyl-amino éthyl méthacrylate (DMAEMA) (quaternisé ou pas) ont été préparés par extrusion.

Les dispersions du copolymère **P18** (ne faisant pas partie de l'objet revendiqué) dans les matériaux solides organiques ont été réalisées avec une mini-extrudeuse bi-vis conique Thermo Scientific (Haake MiniLab II). La température et la vitesse de rotation des vis ont été adaptées à chaque matrice et sont rapportées dans las Tables 6.

**Table 6**

| Matrice Polymérique | Fournisseur | Mw (g/mol) | Température d'Extrusion (°C) | Vitesse de rotation de vis (tr/min) |
|---|---|---|---|---|
| PS | Aldrich | 192000 | 180 | 30 |
| PMMA | Aldrich | 120000 | 200 | 100 |
| PC | TRINSEO | - | 220 | 30 |

Avant l'extrusion, les matériaux solides organiques ont été broyés en fine poudre puis mixés avec 0.5% en poids/matrice du copolymère **P18.** 5 g de chaque mélange a été introduit manuellement dans l'extrudeuse en mode recirculation (1 à 2 min). Apres 2 min en mode recirculation, le mélange a été extrudé en récupéré sous forme d'un jonc.

Des films de matériaux solides organiques ont été préparés à l'aide d'une presse Specac Hydraulic. Les mélanges (joncs) obtenus par extrusion ont été coupés en petits morceaux et puis pressés entre deux disques métalliques recouverts d'un film d'aluminium. La pression a été réglée à 1 tonne et les températures sont les mêmes que celles utilisées en extrusion. Les films obtenus ont une épaisseur moyenne de 330 µm.

### Activités antimicrobiennes d'une série de matériaux solides organiques préparés par extrusion

L'activité antimicrobienne de matériaux solides organiques (ne faisant pas partie de l'objet revendiqué) préparés par extrusion contenant un copolymère dibloc **P18** a été testée. L'activité antimicrobienne a été évaluée selon une procédure adaptée ISO 22196. La matrice polymérique (PS, PMMA, Polycarbonate) contenant le copolymère dibloc est introduite dans des microplaques à 96 puits de polypropylène stériles (Greiner BioOne). Des suspensions bactériennes sont préparées comme réalisé pour les essais CIM, à la différence que la suspension bactérienne en phase logarithmique de croissance (OD600 nm environ 0,6) est centrifugée à 6 000 tr/min pendant 10 min à 4° C. Les culots bactériens sont lavés avec 10 ml de solution saline stérile tamponnée au phosphate (PBS) et centrifugés (6 000 tr/min, 10 min, 4° C). Les bactéries sont finalement diluées dans du PBS stérile pour atteindre une densité bactérienne autour de 10^{E5} bactéries/ml. 10 µl de ces suspensions (correspondant à 1 000 bactéries) sont ajoutés sur la surface de copolymères polymérisés. Des témoins négatifs sont obtenus en ajoutant la suspension bactérienne de 10 µl directement soit dans un puits témoin ne contenant pas de film (échantillon « pas de film », Figure 4), soit sur un film du matériau solide organique extrudé ne contenant pas le copolymère P18 (échantillon « pas de polymère », Figure 4). Après 60 minutes d'incubation à température ambiante, les bactéries sont collectées en ajoutant 90 µl de PBS stérile sur la surface et en répétant le pipetage vers le haut et vers le bas. Les bactéries sont ensuite diluées en série (dilution 1 sur 10) dans du PBS stérile avant d'étaler 10 µl de la suspension bactérienne sur des plaques de gélose LB. Après une nuit d'incubation à 37° C, des plaques sont observées et les colonies sont comptées. Le nombre de bactéries viables a été exprimé en fonction du nombre d'unités de formation de colonies (CFU) observées. Les expériences ont été effectuées en trois exemplaires indépendants (n = 3). Les résultats des tests antimicrobiens effectués sur les matériaux solides organiques préparés par extrusion sont présentés dans la Figure 4.

## Revendications

1. Matériau solide organique comprenant de 0,02 à 2 % en poids d'au moins un copolymère amphiphile à blocs du type méthacrylique, le copolymère à blocs étant dispersé dans une matrice polymérique, le copolymère à blocs présentant une masse molaire moyenne en nombre *(Mn)* supérieure ou égale à 20 000 g/mol, dans lequel le copolymère à blocs comprend au moins un bloc méthacrylique hydrophile comprenant au moins un groupe amine tertiaire et au moins un groupe ion ammonium quaternaire.

2. Matériau solide organique selon la revendication 1, dans lequel le copolymère à blocs présente une masse molaire moyenne en nombre *(Mn)* inférieure ou égale à 100 000 g/mol.

3. Matériau solide organique selon la revendication 1, dans lequel le bloc méthacrylique hydrophile comprend un motif de répétition hydrophile choisi parmi le groupe constitué par le méthacrylate de *N*,*N*-(dialkylamino)alkyle et un ion ammonium quaternaire de celui-ci.

4. Matériau solide organique selon l'une quelconque des revendications précédentes, dans lequel le copolymère à blocs comprend au moins un bloc méthacrylique hydrophobe.

5. Matériau solide organique selon la revendication 4, dans lequel le bloc méthacrylique hydrophobe comprend un motif de répétition hydrophobe choisi parmi le groupe constitué par un méthacrylate d'alkyle linéaire, ramifié, cyclique ou cyclique et ramifié.

6. Matériau solide organique selon la revendication 5, dans lequel le méthacrylate d'alkyle est choisi parmi le groupe constitué par le méthacrylate de méthyle (MMA) et le méthacrylate de butyle (BMA).

7. Matériau solide organique selon l'une quelconque des revendications précédentes, comprenant une des formulations suivantes : PMMA-*b*-PDMAEMA, PMMA-*b*-PDEAEMA, PBMA-*b*-PDMAEMA, PBMA-*b*-PDEAEMA, P(MMA-*Co*-S)-*b*-PDMAEMA, P(MMA-*Co-*S)-*b*-PDEAEMA, P(BMA-*Co*-S)-*b*-PDMAEMA, P(BMA-*Co*-S)-*b*-PDEAEMA, P(MMA-*Co-*ACN)*-b*-PDMAEMA, P(MMA-*Co*-ACN)-*b*-PDEAEMA, P(BMA-*Co*-ACN)-*b*-PDMAEMA, P(BMA-*Co*-ACN)-*b*-PDEAEMA, PMMA-*b*-P(DMAEMA-*Co*-S), PMMA-*b*-P(DEAEMA-*Co*-S), PBMA-*b*-P(DMAEMA-*Co*-S), PBMA-*b*-P(DEAEMA-*Co*-S), PMMA-ib-P(DMAEMA-*Co*-ACN), PMMA-*b*-P(DEAEMA-*Co*-ACN), PBMA-*b*-P(DMAEMA-*Co*-ACN), PBMA-*b-*P(DEAEMA-*Co*-ACN), P(MMA-*Co*-S)-b-P(DMAEMA-*Co*-S), P(MMA-*Co*-S)-*b-*P(DEAEMA-*Co*-S), P(BMA-*Co*-S)-*b*-P(DMAEMA-*Co*-S), P(BMA-*Co*-S)-*b*-P(DEAEMA-*Co*-S), P(MMA-*Co*-ACN)-*b*-P(DMAEMA-*Co*-ACN), P(MMA-*Co*-ACN)-*b*-P(DEAEMA-*Co-*ACN), P(BMA-*Co*-ACN)-*b*-P(DMAEMA-*Co*-ACN), P(BMA-*Co*-ACN)-*b*-P(DEAEMA-*Co-*ACN), P(MMA-*Co*-S)-*b*-P(DMAEMA-*Co*-ACN), P(MMA-*Co*-S)-*b*-P(DEAEMA-*Co*-ACN), P(BMA-*Co*-S)-*b*-P(DMAEMA-*Co*-ACN), P(BMA-*Co*-S)-*b*-P(DEAEMA-*Co*-ACN), P(MMA-*Co*-ACN)-*b*-P(DMAEMA-*Co*-S), P(MMA-*Co*-ACN)-*b*-P(DEAEMA-*Co*-S), P(BMA-*Co*-ACN)-*b*-P(DMAEMA-*Co*-S) et P(BMA-*Co*-ACN)-*b*-P(DEAEMA-*Co*-S).

8. Procédé de préparation d'un matériau solide organique comprenant la dispersion de 0,02 à 2 % en poids d'au moins un copolymère amphiphile à blocs du type méthacrylique dans une matrice polymérique, dans lequel le copolymère à blocs présente une masse molaire moyenne en nombre *(Mn)* supérieure ou égale à 20 000 g/mol, dans lequel le copolymère à blocs comprend au moins un bloc méthacrylique hydrophile comprenant au moins un groupe amine tertiaire et au moins un groupe ion ammonium quaternaire.

9. Procédé selon la revendication 8, comprenant la préparation du copolymère à blocs par polymérisation radicalaire contrôlée par des nitroxydes.

10. Procédé selon la revendication 8 ou la revendication 9, comprenant la quaternisation de l'amine tertiaire.

11. Utilisation d'un matériau solide organique selon l'une quelconque des revendications 1 à 7 pour applications antibactérienne, antimicrobienne, et/ou antifongique non-thérapeutiques.

12. Dispositif médical comprenant un matériau solide organique selon l'une quelconque des revendications 1 à 7.

13. Dispositif médical selon la revendication 12, choisi parmi le groupe constitué par un cathéter.

14. Copolymère amphiphile à blocs du type méthacrylique pour son utilisation en tant que médicament dans un matériau solide organique à une concentration de 0,02 à 2 % en poids, le copolymère à blocs présentant une masse molaire moyenne en nombre *(Mn)* supérieure ou égale à 20 000 g/mol, dans lequel le copolymère à blocs comprend au moins un bloc méthacrylique hydrophile comprenant au moins un groupe amine tertiaire et au moins un groupe ion ammonium quaternaire.

15. Copolymère amphiphile à blocs du type méthacrylique pour son utilisation en tant que médicament antibactérien, antifongique et/ou antiinflammatoire dans un matériau solide organique à une concentration de 0,02 à 2 % en poids, le copolymère à blocs présentant une masse molaire moyenne en nombre *(Mn)* supérieure ou égale à 20 000 g/mol, dans lequel le copolymère à blocs comprend au moins un bloc méthacrylique hydrophile comprenant au moins un groupe amine tertiaire et au moins un groupe ion ammonium quaternaire.

16. Matériau solide organique selon l'une quelconque des revendications 1 à 7 pour son utilisation en tant que médicament antibactérien, antimicrobien, antifongique et/ou antiinflammatoire.

## Patentansprüche

1. Festes organisches Material, das 0,02 bis 2 Gew.-% mindestens eines amphiphilen Blockcopolymers vom Methacryl-Typ umfasst, wobei das Blockcopolymer in einer Polymermatrix dispergiert ist, das Blockcopolymer ein Zahlenmittel der Molmasse (Mn) von größer als oder gleich 20 000 g/mol aufweist, wobei das Blockcopolymer mindestens einen hydrophilen Methacrylblock umfasst, der mindestens eine tertiäre Amingruppe und mindestens eine quaternäre Ammoniumionengruppe umfasst.

2. Festes organisches Material nach Anspruch 1, wobei das Blockcopolymer ein Zahlenmittel der Molmasse (Mn) von kleiner als oder gleich 100 000 g/mol aufweist.

3. Festes organisches Material nach Anspruch 1, wobei der hydrophile Methacrylblock eine hydrophile Repetiereinheit umfasst, die aus der Gruppe bestehend aus einem N,N-(Dialkylamino)alkylmethacrylat und einem quaternären Ammoniumion davon ausgewählt ist.

4. Festes organisches Material nach einem der vorhergehenden Ansprüche, wobei das Blockcopolymer mindestens einen hydrophoben Methacrylblock umfasst.

5. Festes organisches Material nach Anspruch 4, wobei der hydrophobe Methacrylblock eine hydrophobe Repetiereinheit umfasst, die aus der Gruppe bestehend aus einem linearen, verzweigten, cyclischen oder cyclischen und verzweigten Alkylmethacrylat ausgewählt ist.

6. Festes organisches Material nach Anspruch 5, wobei das Alkylmethacrylat ausgewählt ist aus der Gruppe bestehend aus Methylmethacrylat (MMA) und Butylmethacrylat (BMA).

7. Festes organisches Material nach einem der vorhergehenden Ansprüche, das eine der folgenden Formulierungen umfasst: PMMA-b-PDMAEMA, PMMA-b-PDEAEMA, PBMA-b-PDMAEMA, PBMA-b-PDEAEMA, P(MMA-Co-S)-b-PDMAEMA, P(MMA-Co-S)-b-PDEAEMA, P(BMA-Co-S)-b-PDMAEMA, P(BMA-Co-S)-b-PDEAEMA, P(MMA-Co-ACN)-b-PDMAEMA, P(MMA-Co-ACN)-b-PDEAEMA, P(BMA-Co-ACN)-b-PDMAEMA, P(BMA-Co-ACN)-b-PDEAEMA, PMMA-b-P(DMAEMA-Co-S), PMMA-b-P(DEAEMA-Co-S), PBMA-b-P(DMAEMA-Co-S), PBMA-b-P(DEAEMA-Co-S), PMMA-b-P(DMAEMA-Co-ACN), PMMA-b-P(DEAEMA-Co-ACN), PBMA-bP(DMAEMA-Co-ACN), PBMA-b-P(DEAEMA-Co-ACN), P(MMA-Co-S)-bP(DMAEMA-Co-S), P(MMA-Co-S)-b-P(DEAEMA-Co-S), P(BMA-Co-S)-bP(DMAEMA-Co-S), P(BMA-Co-S)-b-P(DEAEMA-Co-S), P(MMA-Co-ACN)-bP(DMAEMA-Co-ACN), P(MMA-Co-ACN)-b-P(DEAEMA-Co-ACN), P(BMA-Co-ACN)-b-P(DMAEMA-Co-ACN), P(BMA-Co-ACN)-b-P(DEAEMA-Co-ACN), P(MMA-Co-S)-b-P(DMAEMA-Co-ACN), P(MMA-Co-S)-b-P(DEAEMA-Co-ACN), P(BMA-Co-S)-bP(DMAEMA-Co-ACN), P(BMA-Co-S)-b-P(DEAEMA-Co-ACN), P(MMA-Co-ACN)-bP(DMAEMA-Co-S), P(MMA-Co-ACN)-b-P(DEAEMA-Co-S), P(BMA-Co-ACN)-bP(DMAEMA-Co-S) und P(BMA-Co-ACN)-b-P(DEAEMA-Co-S).

8. Verfahren zur Herstellung eines festen organischen Materials, welches das Dispergieren von 0,02 bis 2 Gew.-% mindestens eines amphiphilen Blockcopolymers vom Methacryl-Typ in einer Polymermatrix umfasst, wobei das Blockcopolymer ein Zahlenmittel der Molmasse (Mn) von größer als oder gleich 20 000 g/mol aufweist, wobei das Blockcopolymer mindestens einen hydrophilen Methacrylblock umfasst, der mindestens eine tertiäre Amingruppe und mindestens eine quaternäre Ammoniumionengruppe umfasst.

9. Verfahren nach Anspruch 8, das die Herstellung des Blockcopolymers durch eine durch Nitroxide gesteuerte radikalische Polymerisation umfasst.

10. Verfahren nach Anspruch 8 oder Anspruch 9, das die Quaternisierung des tertiären Amins umfasst.

11. Verwendung eines festen organischen Materials nach einem der Ansprüche 1 bis 7 für nichttherapeutische antibakterielle, antimikrobielle und/oder fungizide Anwendungen.

12. Medizinische Vorrichtung, die ein festes organisches Material nach einem der Ansprüche 1 bis 7 umfasst.

13. Medizinische Vorrichtung nach Anspruch 12, die aus der aus einem Katheter bestehenden Gruppe ausgewählt ist.

14. Amphiphiles Blockcopolymer vom Methacryl-Typ zur Verwendung als Medikament in einem festen organischen Material mit einer Konzentration von 0,02 bis 2 Gew.-%, wobei das Blockcopolymer ein Zahlenmittel der Molmasse (Mn) von größer als oder gleich 20 000 g/mol aufweist, wobei das Blockcopolymer mindestens einen hydrophilen Methacrylblock umfasst, der mindestens eine tertiäre Amingruppe und mindestens eine quaternäre Ammoniumionengruppe umfasst.

15. Amphiphiles Blockcopolymer vom Methacryl-Typ zur Verwendung als antibakterielles, fungizides und/oder entzündungshemmendes Medikament in einem festen organischen Material mit einer Konzentration von 0,02 bis 2 Gew.-%, wobei das Blockcopolymer ein Zahlenmittel der Molmasse (Mn) von größer als oder gleich 20 000 g/mol aufweist, wobei das Blockcopolymer mindestens einen hydrophilen Methacrylblock umfasst, der mindestens eine tertiäre Amingruppe und mindestens eine quaternäre Ammoniumionengruppe umfasst.

16. Festes organisches Material nach einem der Ansprüche 1 bis 7 zur Verwendung als antibakterielles, antimikrobielles, fungizides und/oder entzündungshemmendes Medikament.

## Claims

1. A solid organic material comprising from 0.02 to 2 wt% of at least one amphiphilic block copolymer of the methacrylic type, the block copolymer being dispersed in a polymer matrix, the block copolymer having a number-average molar mass (*Mn*) greater than or equal to 20 000 g/mol, wherein the block copolymer comprises at least one hydrophilic methacrylic block comprising at least one tertiary amine group and at least one quaternary ammonium ion.

2. The solid organic material as claimed in claim 1, wherein the block copolymer has a number-average molar mass (*Mn*) less than or equal to 100 000 g/mol.

3. The solid organic material as claimed in claim 1, wherein the hydrophilic methacrylic block comprises a hydrophilic repeating unit selected from the group consisting of *N,N*-(dialkylamino)alkyl methacrylate and a quaternary ammonium ion of the latter.

4. The solid organic material as claimed in any one of the preceding claims, wherein the block copolymer comprises at least one hydrophobic methacrylic block.

5. The solid organic material as claimed in claim 4, wherein the hydrophobic methacrylic block comprises a hydrophobic repeating unit selected from the group consisting of a linear, branched, cyclic or cyclic and branched alkyl methacrylate.

6. The solid organic material as claimed in claim 5, wherein the alkyl methacrylate is selected from the group consisting of methyl methacrylate (MMA) and butyl methacrylate (BMA).

7. The solid organic material as claimed in any one of the preceding claims, comprising one of the following formulations: PMMA-*b*-PDMAEMA, PMMA-*b*-PDEAEMA, PBMA-*b*-PDMAEMA, PBMA-*b*-PDEAEMA, P(MMA-*Co*-S)-b-PDMAEMA, P(MMA-*Co*S)-*b*-PDEAEMA, P(BMA-*Co*-S)-*b*-PDMAEMA, P(BMA-*Co*-S)-b-PDEAEMA, P(MMA-*Co-*ACN)-*b*-PDMAEMA, P(MMA-*Co*-ACN)-*b*-PDEAEMA, P(BMA-*Co*-ACN)-*b*-PDMAEMA, P(BMA-*Co*-ACN)-*b*-PDEAEMA, PMMA-b-P(DMAEMA-*Co*-S), PMMA-*b*-P(DEAEMA-*Co*-S), PBMA-*b*-P(DMAEMA-*Co*-S), PBMA-*b*-P(DEAEMA-*Co*-S), PMMA-*b*-P(DMAEMA-*Co*-ACN), PMMA-*b*-P(DEAEMA-*Co*-ACN), PBMA-*b*-P(DMAEMA-*Co*-ACN), PBMA-*b-*P(DEAEMA-*Co*-ACN), P(MMA-*Co*-S)-*b*-P(DMAEMA-*Co*-S), P(MMA-*Co*-S)-*b-*P(DEAEMA-*Co*-S), P(BMA-*Co*-S)-*b*-P(DMAEMA-Co-S), P(BMA-*Co*-S)-*b*-P(DEAEMA-*Co*-S), P(MMA-*Co*-ACN)-*b*-P(DMAEMA-*Co*-ACN), P(MMA-*Co*-ACN)-*b*-P(DEAEMA-*Co-*ACN), P(BMA-*Co*-ACN)-*b*-P(DMAEMA-*Co*-ACN), P(BMA-*Co-*ACN)-*b*-P(DEAEMA-*Co-*ACN), P(MMA-*Co*-S)-*b*-P(DMAEMA-*Co*-ACN), P(MMA-*Co*-S)-*b*-P(DEAEMA-*Co*-ACN), P(BMA-*Co*-S)-*b*-P(DMAEMA-*Co*-ACN), P(BMA-*Co*-S)-*b*-P(DEAEMA-*Co*-ACN), P(MMA-*Co*-ACN)-*b*-P(DMAEMA-*Co*-S), P(MMA-*Co*-ACN)-*b*-P(DEAEMA-*Co*-S), P(BMA-*Co*-ACN)-*b*-P(DMAEMA-*Co*-S) and P(BMA-*Co*-ACN)-*b*-P(DEAEMA-*Co*-S).

8. A method for preparing a solid organic material comprising dispersing from 0.02 to 2 wt% of at least one amphiphilic block copolymer of the methacrylic type in a polymer matrix, wherein the block copolymer has a number-average molar mass *(Mn)* greater than or equal to 20 000 g/mol, wherein the block copolymer comprises at least one hydrophilic methacrylic block comprising at least one tertiary amine group and at least one quaternary ammonium ion.

9. The method as claimed in claim 8, comprising preparing the block copolymer by nitroxide-mediated radical polymerization.

10. The method as claimed in claim 8 or claim 9, comprising quaternization of the tertiary amine.

11. Use of the solid organic material as claimed in any one of claims 1 to 7 for nontherapeutic antibacterial, antimicrobial, and/or antifungal applications.

12. A medical device comprising a solid organic material as claimed in any one of claims 1 to 7.

13. The medical device as claimed in claim 12, selected from the group consisting of a catheter.

14. An amphiphilic block copolymer of the methacrylic type for use thereof as a medicament in a solid organic material at a concentration of from 0.02 to 2 wt%, the block copolymer having a number-average molar mass *(Mn)* greater than or equal to 20 000 g/mol, wherein the block copolymer comprises at least one hydrophilic methacrylic block comprising at least one tertiary amine group and at least one quaternary ammonium ion.

15. An amphiphilic block copolymer of the methacrylic type for use thereof as an antibacterial, antifungal and/or anti-inflammatory medicament in a solid organic material at a concentration of from 0.02 to 2 wt%, the block copolymer having a number-average molar mass *(Mn)* greater than or equal to 20 000 g/mol, wherein the block copolymer comprises at least one hydrophilic methacrylic block comprising at least one tertiary amine group and at least one quaternary ammonium ion.

16. The solid organic material as claimed in any one of claims 1 to 7 for use as an antibacterial, antimicrobial, antifungal and/or anti-inflammatory medicament.
